# EUROPEAN PATENT SPECIFICATION

(11) **EP 0 440 037 B1**
(45) Date of publication and mention of the grant of the patent: **09.08.1995**
(21) Application number: 91100407.5
(22) Date of filing: 15.01.1991
(51) Int. Cl.: C07C 403/24

(54) **Hydroxycarbonyl derivatives and process for making the same**
Hydroxycarbonylderivate und Verfahren zu ihrer Herstellung
Dérivés hydroxycarbonylés et procédé pour leur préparation

(30) Priority: 01.02.1990 US 473352
(43) Date of publication of application: 07.08.1991
(73) Proprietor: NEUROSEARCH A/S, DK-2600 Glostrup (DK)
(72) Inventor: Moldt, Peter, DK-3050 Humlebaek (DK)
(74) Representative: Grünecker, Kinkeldey, Stockmair & Schwanhäusser Anwaltssozietät

(56) References cited:
- EP-A- 0 101 597
- THE JOURNAL OF ORGANIC CHEMISTRY, vol. 49, 1984, F.A. DAVIS et al. "Synthesis of alpha-Hydroxy Carbonyl Compounds (Acyloins) . Diorect Oxidation of Enolates 2-Sulfonyloxaziridines" pages 3241-3243.

## Description

The present invention relates to a novel process for the manufacture of hydroxycyclohexenone derivatives, more specifically to the manufacture of astaxanthin as well as to a novel astaxanthin dihemiaminal. Astaxanthin is widely used and has important value as an additive in the fish industry.

It has long been known that astaxanthin can be prepared from canthaxanthin in low yield via astacin and crustaxanthin. J. Chem. Soc. Chem. Commun. 49 (1967). This process is not suitable for commercial purposes.

From US-A-4,585,885 it is known that astaxanthin can be prepared in a four-step synthetic sequence from canthaxanthin. The process is indicated to be of commercial value. However, it is apparent that also this process is rather laborious and results in considerable losses and low yields of product.

The reaction sequence as described in US-A-4,585,885 involves the formation of either an alkylether or a trialkylsilylether as being necessary to protect the initially-formed metal enolate from destruction during the following oxidation reaction which involves the application of an acidic oxidant.

It has recently been described (J. Org. Chem. 49, 3241-3243 (1984)) that trans-2-(phenylsulfonyl)-3-phenyloxaziridine can oxidize certain metal enolates. This oxidizing agent is also known to epoxidize alkene double bonds. Tetrahedron Letters, page 917 (1981).

It is also a well-known fact that carotenoid compounds are extremely sensitive to oxygen and other oxidizing agents, the result being a variety of products rather than any single desired end product. Accordingly, until the present invention, it was not known what effect such an oxidizing agent might have upon the extremely sensitive carotenoid compounds, and especially upon a canthaxanthin compound, particularly a canthaxanthin dienolate, which is employed as an intermediate according to the method of the present invention.

It has now surprisingly been found that astaxanthin can be prepared in a very simple and selective way from canthaxanthin by oxidizing a metal dienolate of canthaxanthin with an oxaziridine oxidant.

The best known method for the production of astaxanthin up until the time of the present invention appears to be that of US-A-4,585,885, issued April 29, 1986, which produces astaxanthin by a reaction sequence involving four steps, namely, preparation of a lithium enolate, preparation of an alkylsilyl enolether from the lithium enolate, oxidation of the alkylsilyl enolether using a percarboxylic acid, and finally removal of the protecting alkylsilyl groups by hydrolysis. The process of the present invention involves entirely different intermediates than the previous process and does not involve the employment of an alkylsilyl protecting group and removal thereof, consequently shortening the process by the elimination of two steps, thereby rendering the present process more economically efficient, especially since it is conveniently conducted as a single-pot reaction. Moreover, the oxidant employed in the process of the present invention, upon completion of the reaction, is in the form of a compound which is in fact a one-step removed starting material for preparation of the oxidant itself, thereby providing the opportunity for even greater economy by simply recycling the conversion product of the oxidant employed back to produce additional starting oxidant.

It is the object of the present invention to provide a novel method for preparing astaxanthin from canthaxanthin wherein the enolate of canthaxanthin is oxidized without the use of protective groups, as well as to provide key intermediates in said process.

According to the present invention this is obtained with a process for the preparation of astaxanthin having the formula 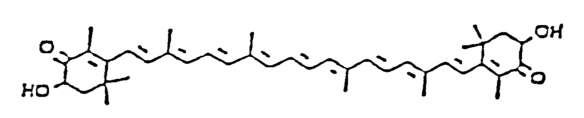
comprising the steps of:
treating canthaxanthin with an alkali metal base to produce a canthaxanthin alkali metal dienolate of the formula 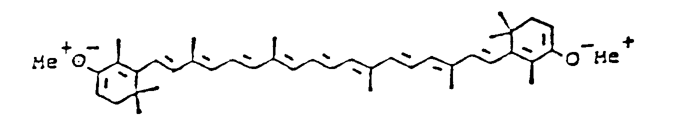
wherein
Me is an alkali metal,
treating the canthaxanthin alkali metal dienolate with a compound having the formula 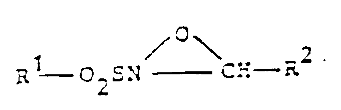
wherein R¹ is phenyl, phenyl substituted with a substituent which is stable under the conditions of reaction, C₃₋₇-cycloalkyl, camphoryl, or another cyclic or bicyclic radical which may carry alkyl, keto, or other substituents which are stable under the conditions of reaction, and wherein R² independently signifies hydrogen or the same radical as R¹, or wherein R¹ and R² together form a cyclic or bicyclic radical;
to produce an astaxanthin dihemiaminal having the formula 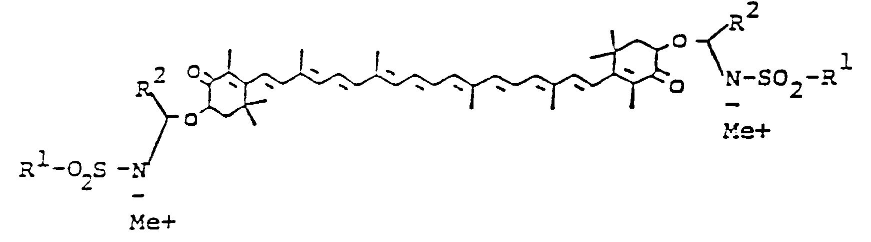
wherein R¹, R² and Me are as defined above,
and decomposing the astaxanthin dihemiaminal to obtain the astaxanthin.

In a preferred process both R¹ and R² are phenyl or camphoryl.

Moreover, the present object of providing key intermediates is achieved with an astaxanthin dihemiaminal having the formula 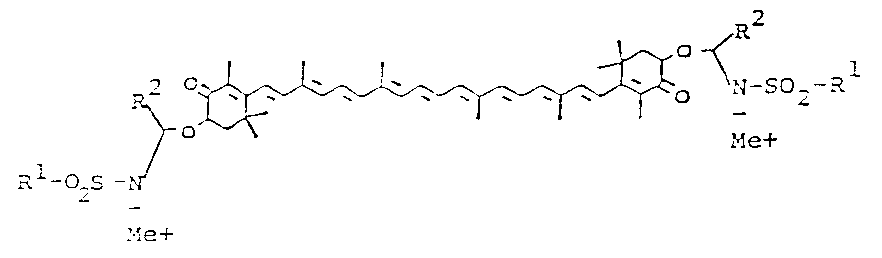
wherein R¹ is phenyl, phenyl substituted with a substituent which is stable under the conditions of the process defined in claim 1, C₃₋₇-cycloalkyl, camphoryl, or another cyclic or bicyclic radical which may carry alkyl, keto, or other substituents which are stable under the conditions of the process defined in claim 1, and wherein R² independently signifies hydrogen or the same radical as R¹, or wherein R¹ and R² together form a cyclic or bicyclic radical, and Me is an alkali metal.

A compound wherein both R¹ and R² are phenyl or wherein R¹ and R² together are camphoryl is preferred. The present invention also provides a composition containing such a compound in an organic solvent; and such a composition wherein the solvent is tetrahydrofuran, dioxane, or an aromatic solvent.

It is further preferred that the oxidation in the present process is carried out in the presence of an organic solvent which is nonreactive with the reactants and reaction products under the conditions of reaction. Preferably said solvent is tetrahydrofuran, dioxane, or an aromatic solvent. Further, preferably the oxidation is carried out at a temperature between -78°C and -20°C; and the oxidation is preferably carried out in an inert atmosphere. Further preferred is a process wherein the decomposition is carried out with a proton donor at a temperature up to 30°C, the proton donor preferably being acetic acid or ammonium chloride. When both R¹ and R² are phenyl, it is preferable that the process is carried out in the presence of an organic solvent which is non-reactive with the reactants and reaction products under the conditions of reaction and at a temperature up to 30°C and employing a proton donor.

When R¹ and R² are both phenyl or together are camphoryl, then the decomposition is preferably carried out in the presence of an organic solvent which is non-reactive with the reactants and reaction products under the conditions of reaction at a temperature up to 30°C and in the presence of a proton donor, and wherein the oxidation is carried out in the presence of an inert gas at a temperature between -78°C and -20°C and in the presence of an organic solvent which is non-reactive with the reactants and reaction products under the conditions of reaction Finally, such a process wherein an excess of the oxidant is employed, is preferred.

As already stated, according to the process provided by the present invention, astaxanthin is accessible in fewer steps from canthaxanthin than known before, e.g., in US-A-4,585,885.

The following scheme illustrates the novel process of the invention, which is conveniently carried out as a one-pot reaction: 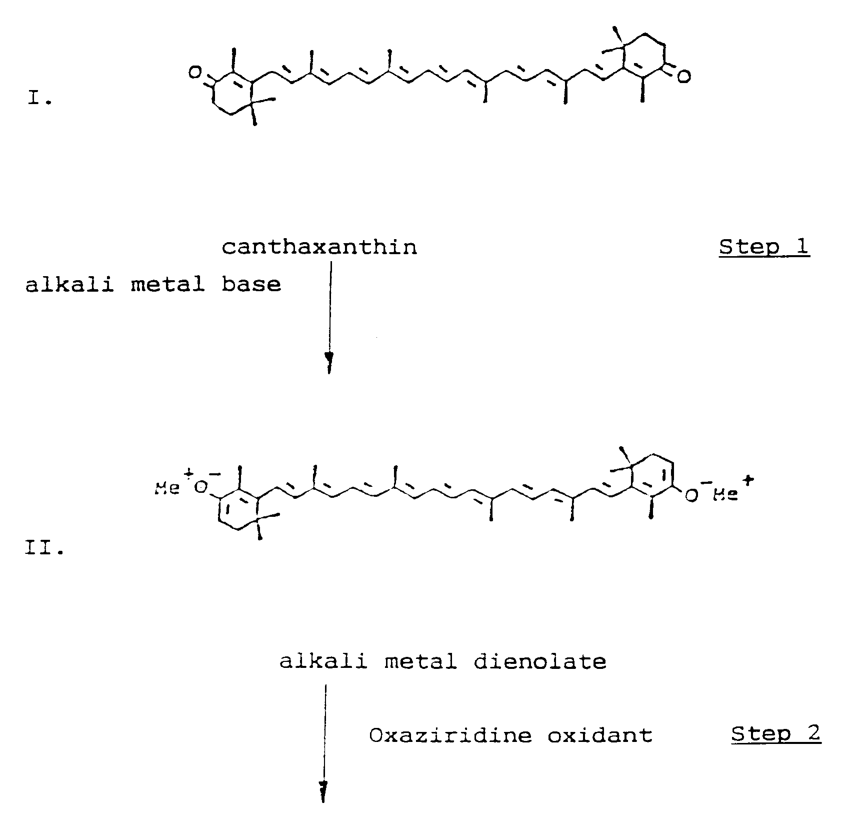 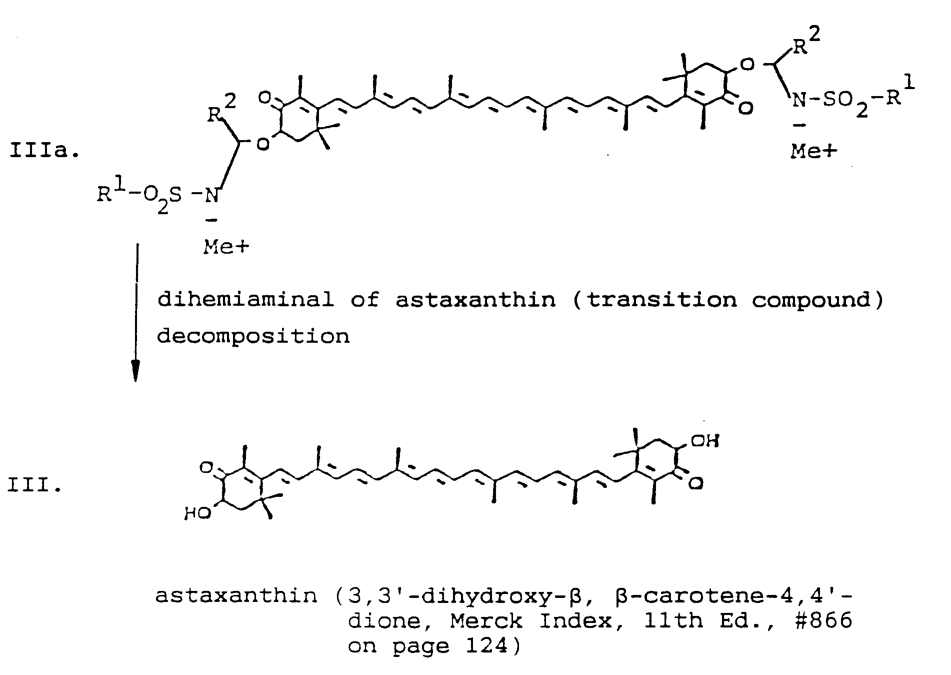
wherein R¹ and R² are as defined hereinafter.

The reactions of the invention as illustrated above are carried out in an organic solvent, which is not critical. Preferred organic solvents or mixtures thereof are selected from ethers, especially cyclic ethers such as for example tetrahydrofuran (THF) and dioxane, aromatic solvents such as for example benzene, toluene and xylene. Among other suitable solvents glymes and dimethylformamide (DMF) can be mentioned. THF and toluene are especially suitable solvents.

Among preferred alkali metal bases for the preparation of the initially-formed metal enolate in step 1 of the above scheme, lithium diisopropylamide and an alkalimetal bis(trimethylsilyl)amide can be mentioned. Other strong alkalimetal bases can be used instead. For example, sodium hydride, potassium hydride, and potassium t-butanolate are suitable. It has been found that sodium bis(trimethylsilyl)amide is especially suitable for the preparation of a reactive canthaxanthin dialkalimetal enolate in step 1 of the above scheme.

The decomposition step as illustrated in the foregoing scheme is an inevitable consequence following the preparation of the dihemiaminal compound of astaxanthin. The decomposition reaction takes place either as a decomposition of the alkalimetal dihemiaminal itself leading to the astaxanthin dialkalimetal alcoholate (which is then quenched to give astaxanthin) and a sulfonimine or the alkalimetal dihemiaminal is protonated by addition of the quenching agent and then the dihemiaminal decomposes to give astaxanthin.

The quenching agent employed according to the invention can be any proton donor. Therefore any protic compound may be employed. Among protic compounds water, alcohols, and hydrogen-donating acids can be mentioned as representative. It is preferred that the quenching proton donor is at least slightly acidic in order to avoid the formation of strong base during the quenching step.

It is preferred to carry out the preparation of astaxanthin from canthaxanthin as a one-pot reaction.

In a preferred procedure for the preparation of astaxanthin according to the invention, canthaxanthin is dissolved in an organic solvent and the reaction conducted at reduced temperature under an inert atmosphere. For example, when the solvent is THF and the inert atmosphere is nitrogen, the temperature may be from -78°C to -20°C. The alkalimetal base in an organic solvent is then added dropwise to the canthaxanthin solution to prepare the alkalimetal dienolate. Then the selected oxidant in an organic solvent is added directly to the already-prepared mixture of canthaxanthin and alkalimetal base, comprising the alkalimetal dienolate in solution or suspension. After decomposition, the desired astaxanthin can be isolated directly by usual techniques.

In another much preferred procedure for carrying out the method of the present invention, the oxidant and canthaxanthin are mixed in an organic solvent or mixture of organic solvents at a reduced temperature under an inert atmosphere. Thereafter the alkalimetal base in an organic solvent is added incrementally to effect the incremental conversion to the alkalimetal dienolate and its concurrent or simultaneous oxidation to astaxanthin. This method is especially suitable for large-scale manufacture of astaxanthin inasmuch as it avoids precipitation of the intermediate alkalimetal dienolate.

In order to effect the reactions of the invention, it has been found advantageous to employ slight excesses of the alkalimetal base and of the oxidant. For example, it has been found that 1.2 equivalents of the base and 1.3 equivalents of the oxidant give excellent yields of the end product (astaxanthin). Equivalent or near equivalent amounts of base and oxidant will, however, still give satisfactory amounts of the desired end product.

The oxaziridine oxidants, as employed according to the present invention, are known and have the formula 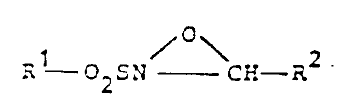
wherein the R¹ radical is phenyl, phenyl substituted with a substituent which is stable under the conditions of reaction, e.g., phenyl substituted with halogen, nitro, cyano, C₁₋₄-alkyl or C₁₋₄-alkoxy; C₃₋₇-cycloalkyl; or camphoryl, or another cyclic or bicyclic radical which may carry alkyl, keto, or other substituents which are stable under the conditions of reaction, and R² independently signifies hydrogen or the same radical as R¹, or wherein R¹ and R² together form a cyclic or bicyclic radical.

In order to reduce the production costs of astaxanthin when prepared according to the invention, it is preferred to utilize an oxidant as defined above wherein R¹ and R² are both phenyl. This oxidant gives excellent yields of astaxanthin when prepared from canthaxanthin in the manner of the invention.

The high yields and purity of astaxanthin according to the present invention (in Example 1, 71% isolated yield with greater than 95% purity) compare most favourably with the unisolated yields according to the procedure of US-A-4,585,885 (Column 8) of 67.5% and the isolated yield of 48% astaxanthin of about 90% purity (Column 10, line 19 thereof).

The invention will now be described in greater detail with reference to the following examples.

### Example 1

2.82 g (5 mmol) canthaxanthin (from Fluka, Basel, Switzerland, used without prior purification) was dissolved in 150 ml absolute tetrahydrofuran and cooled to -20°C under nitrogen. 12 ml of 1M (in tetrahydrofuran) solution of sodium-hexamethyl-disilazane (12 mmol) was added over a period of 5 min, and the heterogeneous mixture was stirred for 30 min and cooled to -78°C to give the alkalimetal dienolate. A solution of 3.40 g (13 mmol) trans-2-(phenylsulfonyl)-3-phenyloxaziridine in 30 ml of absolute tetrahydrofuran was added within 5 min and the mixture was stirred at -78°C for an additional 30 min to give the dihemiaminal of astaxanthin, which probably subsequently decomposes to give a sulphonimine and the astaxanthindienolate anion. The reaction was then quenched by addition of 698 »l (12.2 mmol) of glacial acetic acid, and was allowed to reach O°C. After evaporation in vacuo at 30°C, the crude reaction product was chromatographed on silica gel with methylene chloride/diethyl ether (9:1) as the eluent. The fraction containing astaxanthin was concentrated in vacuo yielding astaxanthin as violet crystals. The astaxanthin was dissolved in a minimum amount of methylene chloride, precipitated with pentane, and 2.12 g (71% based on starting canthaxanthin) of violet crystals were collected by filtration. Purity estimated by TLC [diethyl ether/pentane (2:1)] was greater than 95%; identified by comparison by ¹³C-NMR with an, authentic sample of astaxanthin.

### Example 2 (Oxidation of Alkalimetal Dienolate as Formed in situ)

113 mg (0.2 mmol) canthaxanthin and 157 mg (0.6 mmol) trans-2-(phenylsulfonyl)-3-phenyloxaziridine was dissolved in 10 ml absolute tetrahydrofuran and cooled to -78°C under nitrogen. 600 »l of a 1M solution (0.6 mmol) of sodium-hexamethyl-disilazane was added over a period of 20 min, and the mixture was stirred for an additional 20 min, to give the astaxanthin dihemiaminal by oxidation of the canthaxanthin alkalimetal dienolate as it was formed in situ. The reaction mixture was quenched by addition of 46 »l (0.8 mmol) glacial acetic acid and the yield of astaxanthin was estimated by TLC diethyl ether/pentane (2:1) to be 20% based on starting canthaxanthin.

### Example 3 (Different Solvent)

565 mg (1 mmol) canthaxanthin was dissolved in 60 ml absolute toluene and cooled to -1O°C under nitrogen. 3 ml of a 1M (in tetrahydrofuran) solution of sodium-hexamethyl-disilazane (3 mmol) was added over a period of 2 min, and the heterogeneous mixture was stirred for 60 min and cooled to -78°C to produce the alkalimetal dienolate. A solution of 800 mg (3 mmol) trans-2-(phenylsulfonyl)-3-phenyloxaziridine in 20 ml of absolute toluene was added within 5 min and the mixture was stirred at -78°C for an additional 30 min to produce the astaxanthin dihemiaminal, which probably subsequently decomposes to give a sulphonimine and the astaxanthindienolate anion. The reaction mixture was quenched by addition of 172 »l (3 mmol) glacial acetic acid, and was allowed to reach O°C. After evaporation in vacuo at 30°C, the crude reaction product was chromatographed on silica gel with methylene chloride/diethyl ether (9:1) as the eluent. The fraction containing astaxanthin was concentrated in vacuo yielding 80 mg (13%) astaxanthin as violet crystals.

### Example 4 (Different Base and Quenching Agent)

1 g (1.77 mmol) canthaxanthin was dissolved in 20 ml absolute tetrahydrofuran and cooled to -1O°C under nitrogen. 10.6 ml of 0.5M (in toluene) solution of potassium-hexamethyl-disilazane (5.31 mmol) was added over a period of 5 min, and the heterogeneous mixture was stirred for 30 min and cooled to -78°C to prepare the alkalimetal dienolate. A solution of 1.39 g (5.31 mmol) trans-2-(phenylsulfonyl)-3-phenyloxaziridine in 20 ml of absolute tetrahydrofuran was added within 5 min and the mixture was stirred at -78°C for an additional 30 min to prepare the astaxanthin dihemiaminal, which probably subsequently decomposes to give a sulphonimine and the astaxanthindienolate anion. The reaction mixture was quenched by addition of 4 ml saturated ammoniumchloride solution and was allowed to reach O°C. The tetrahydrofuran was removed on a rotary evaporator at 30°C, 10 ml of water was added, and the mixture was extracted three times with 20 ml methylenechloride. The combined organic phases were washed with 10 ml of brine, dried over sodium sulphate, and concentrated in vacuo at 30°C. The crude reaction product was chromatographed on silica gel with methylene chloride/diethyl ether diethyl ether (9:1) as the eluent. The fractions containing astaxanthin were concentrated in vacuo yielding 82 mg (8%) astaxanthin as violet crystals.

### Example 5 (Use of Lithium dienolate)

In the same manner as given in Example 1, the process is carried out employing lithium-hexamethyl-disilazane instead of sodium-hexamethyl-disilazane with essentially the same result.

### Example 6 (Different Oxidizing Agent)

In the same manner as given in Example 1, the process is repeated, only using instead of the trans-2-(phenylsulfonyl)-3-phenyloxaziridine the bicyclic oxidizing agent (+)-(2R,8aS)-camphorylsulfonyloxaziridine. The result is essentially the same as in Example 1.

### Example 7 (Different Solvent)

The procedure of Example 1 is repeated exactly with the exception of the fact that dioxane is substituted as solvent for the tetrahydrofuran. The results are essentially the same as given in Example 1.

### Example 8

### Astaxanthin (oxidation using camphoryl-oxaziridine)

1.41 g (2.5 mmol) canthaxanthin was dissolved in 200 ml absolute tetrahydrofuran and cooled to -20°C under argon. 6 ml of 1M (in tetrahydrofuran) solution of sodium-hexamethyl-disilazane (6 mmol) was added over a period of 5 min, and the mixture was stirred for 30 min and cooled to -78°C. A solution of 1.5 g (6.5 mmol) (+)-(2R,8aS)-10-(camphorylsulfonyl)oxaziridine (prepared as described in Organic Synthesis 69, 158-168 (1990)) in 30 ml absolute tetrahydrofuran was added within 5 min and the mixture was stirred for an additional 60 min. The reaction mixture was quenched by addition of 0.35 ml glacial acetic acid and the mixture was concentrated in vacuo. The remanescens was triturated with 25 ml methanol and left at 4°C overnight, and the formed crystals were filtered off. The crude product was chromatographed on silica gel with methylene chloride/diethyl ether (9:1) as the eluent. The fractions containing astaxanthin was concentrated in vacuo yielding 0.68 g (46% based on canthaxanthin) astaxanthin as violet crystals. The optical purity of the astaxanthin was determined by HPLC analysis of the (-)camphanic acid esters of astaxanthin as described in Journal of High Resolution Chromatography & Chromatography Communications 2, 195-196 (1979), and the isomer distribution was 15% (3S, 3'S), 49% (3S, 3'R) and 36% (3R, 3'R).

It is thus seen that the present invention provides a new and economic process for the production of astaxanthin from canthaxanthin according to procedure which permits the elimination of two steps, that is, by a process which involves two steps less than the best known prior art process, involving the employment of novel intermediates and the application of novel oxidizing agents to the said novel intermediates, resulting in production of the highly desirable astaxanthin product, which is in great demand in the fish industry and in the food industry generally for the colouring of food products, in high yields and purity, thus fulfilling a technological and economic demand for a more efficient and simple process, especially since the process of the present invention is well adapted to and preferably conducted as a one-pot reaction.

## Claims (Claims for the following Contracting State(s): AT, BE, CH, DE, DK, FR, GB, IT, LI, LU, NL, SE)

1. A process for the preparation of astaxanthin having the formula 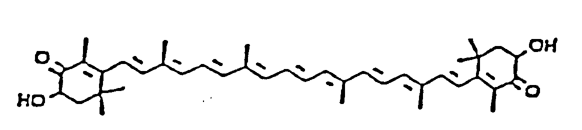 comprising the steps of:
treating canthaxanthin with an alkali metal base to produce a canthaxanthin alkali metal dienolate of the formula 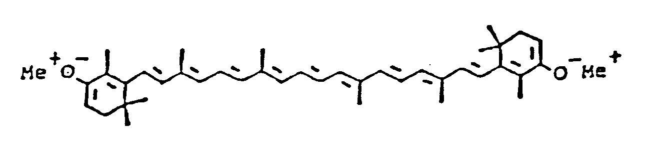 wherein
Me is an alkali metal,
treating the canthaxanthin alkali metal dienolate with a compound having the formula 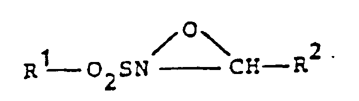 wherein R¹ is phenyl, phenyl substituted with a substituent which is stable under the conditions of reaction, C₃₋₇-cycloalkyl, camphoryl, or another cyclic or bicyclic radical which may carry alkyl, keto, or other substituents which are stable under the conditions of reaction, and wherein R² independently signifies hydrogen or the same radical as R¹, or wherein R¹ and R² together form a cyclic or bicyclic radical;
to produce an astaxanthin dihemiaminal having the formula 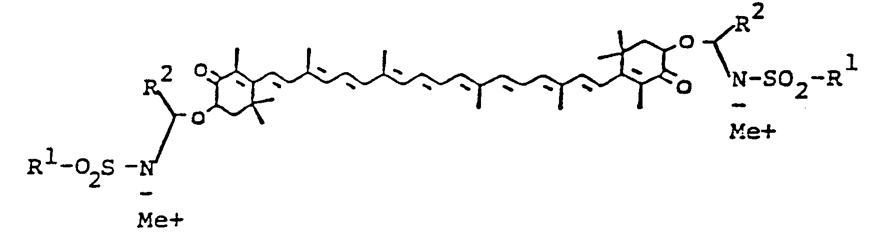 wherein R¹, R² and Me are as defined above,
and decomposing the astaxanthin dihemiaminal to obtain the astaxanthin.

2. The process of claim 1, wherein both R¹ and R² are phenyl.

3. The process of claim 1, wherein R¹ and R² together are camphoryl.

4. The process of claim 1, wherein the oxidation is carried out in the presence of an organic solvent which is nonreactive with the reactants and reaction products under the conditions of reaction.

5. The process of claim 4, wherein the solvent is tetrahydrofuran, dioxane, or an aromatic solvent.

6. The process of claim 4, wherein the oxidation is carried out at a temperature between -78°C and -20°C.

7. The process of claim 1, wherein the oxidation is carried out in an inert atmosphere.

8. The process of claim 1, wherein decomposition is carried out with a proton donor at a temperature up to 30°C.

9. The process of claim 2, wherein the decomposition is carried out in the presence of an organic solvent which is non-reactive with the reactants and reaction products under the conditions of reaction and at a temperature up to 30°C and employing a proton donor.

10. The process of claim 8, wherein the proton donor is acetic acid or ammonium chloride.

11. The process of claim 2 or 3, wherein the decomposition is carried out in the presence of an organic solvent which is non-reactive with the reactants and reaction products under the conditions of reaction at a temperature up to 30°C and in the presence of a proton donor, and wherein the oxidation is carried out in the presence of an inert gas at a temperature between -78°C and -20°C and in the presence of an organic solvent which is non-reactive with the reactants and reaction products under the conditions of reaction.

12. The process of claim 1, wherein an excess of the oxidant is employed.

13. An astaxanthin dihemiaminal having the formula 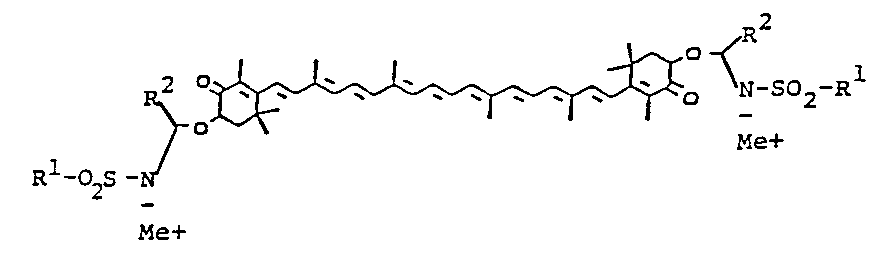 wherein R¹ is phenyl, phenyl substituted with a substituent which is stable under the conditions of the process defined in claim 1, C₃₋₇-cycloalkyl, camphoryl, or another cyclic or bicyclic radical which may carry alkyl, keto, or other substituents which are stable under the conditions of the process defined in claim 1, and wherein R² independently signifies hydrogen or the same radical as R¹, or wherein R¹ and R² together form a cyclic or bicyclic radical, and Me is an alkali metal.

14. A compound of claim 13, wherein both R¹ and R² are phenyl.

15. A compound of claim 13, wherein R¹ and R² together are camphoryl.

16. A composition containing a compound of any of claims 13 to 15, in an organic solvent.

17. The composition of claim 16, wherein the solvent is tetrahydrofuran, dioxane, or an aromatic solvent.

## Claims (Claims for the following Contracting State(s): ES)

1. A process for the preparation of astaxanthin having the formula 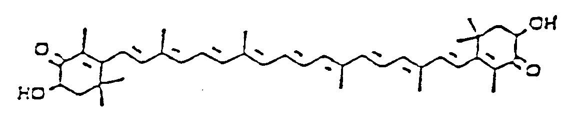 comprising the steps of:
treating canthaxanthin with an alkali metal base to produce a canthaxanthin alkali metal dienolate of the formula 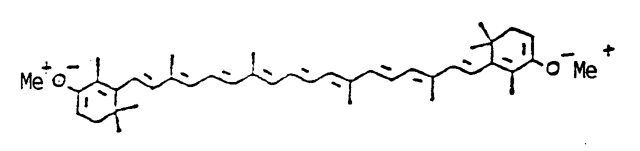 wherein
Me is an alkali metal,
treating the canthaxanthin alkali metal dienolate with a compound having the formula 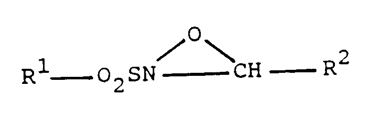 wherein R¹ is phenyl, phenyl substituted with a substituent which is stable under the conditions of substituent which is stable under the conditions of reaction, C₃₋₇-cycloalkyl, camphoryl, or another cyclic or bicyclic radical which may carry alkyl, keto, or other substituents which are stable under the conditions of reaction, and wherein R² independently signifies hydrogen or the same radical as R¹, or wherein R¹ and R² together form a cyclic or bicyclic radical;
to produce an astaxanthin dihemiaminal having the formula 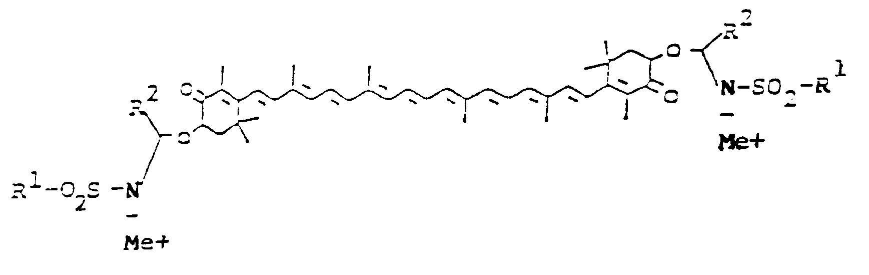 wherein R¹, R² and Me are as defined above,
and decomposing the astaxanthin dihemiaminal to obtain the astaxanthin.

2. The process of claim 1, wherein both R¹ and R² are phenyl.

3. The process of claim 1, wherein R¹ and R² together are camphoryl.

4. The process of claim 1, wherein the oxidation is carried out in the presence of an organic solvent which is nonreactive with the reactants and reaction products under the conditions of reaction.

5. The process of claim 4, wherein the solvent is tetrahydrofuran, dioxane, or an aromatic solvent.

6. The process of claim 4, wherein the oxidation is carried out at a temperature between -78°C and -20°C.

7. The process of claim 1, wherein the oxidation is carried out in an inert atmosphere.

8. The process of claim 1, wherein decomposition is carried out with a proton donor at a temperature up to 30°C.

9. The process of claim 2, wherein the decomposition is carried out in the presence of an organic solvent which is non-reactive with the reactants and reaction products under the conditions of reaction and at a temperature up to 30°C and employing a proton donor.

10. The process of claim 8, wherein the proton donor is acetic acid or ammonium chloride.

11. The process of claim 2 or 3, wherein the decomposition is carried out in the presence of an organic solvent which is non-reactive with the reactants and reaction products under the conditions of reaction at a temperature up to 30°C and in the presence of a proton donor, and wherein the oxidation is carried out in the presence of an inert gas at a temperature between -78°C and -20°C and in the presence of an organic solvent which is non-reactive with the reactants and reaction products under the conditions of reaction.

12. The process of claim 1, wherein an excess of the oxidant is employed.

13. A process for preparing an astaxanthin dihemiaminal having the formula 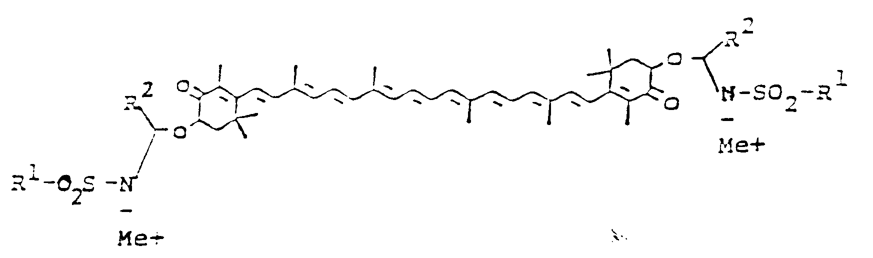 wherein R¹ is phenyl, phenyl substituted with a substituent which is stable under the conditions of the process defined in claim 1, C₃₋₇-cycloalkyl, camphoryl, or another cyclic or bicyclic radical which may carry alkyl, keto or other substituents which are stable under the conditions of the process defined in claim 1, and wherein R² independently signifies hydrogen or the same radical as R¹, or wherein R¹ and R² together form a cyclic or bicyclic radical, and Me is an alkali metal,
comprising the steps of:
treating canthaxanthin with an alkali metal base to produce a canthaxanthin alkali metal dienolate of the formula 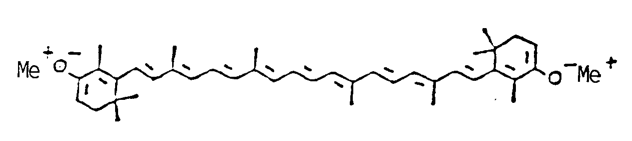 wherein
Me is as defined above, and
treating the canthaxanthin alkali metal dienolate with a compound having the formula 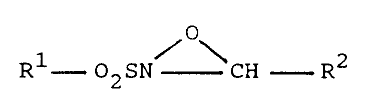 wherein R¹ and R² are defined as above to produce the astaxanthin dihemiaminal.

14. The process of claim 13, wherein both R¹ and R² are phenyl.

15. A compound of claim 13, wherein R¹ and R² together are camphoryl.

16. A process for preparing a composition containing a compound prepared according to any of claims 13 to 15 comprising mixing said compound and an organic solvent.

17. The process of claim 16, wherein the solvent is tetrahydrofuran, dioxane, or an aromatic solvent.

## Claims (Claims for the following Contracting State(s): GR)

1. A process for the preparation of astaxanthin having the formula 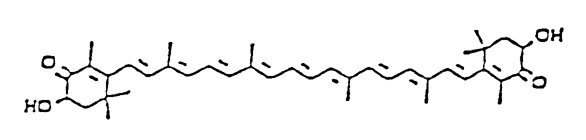 comprising the steps of:
treating canthaxanthin with an alkali metal base to produce a canthaxanthin alkali metal dienolate of the formula 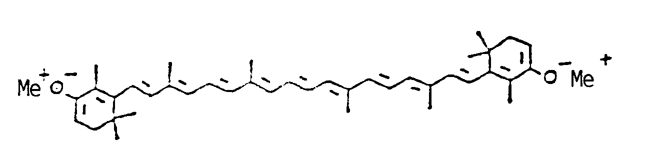 wherein
Me is an alkali metal,
treating the canthaxanthin alkali metal dienolate with a compound having the formula 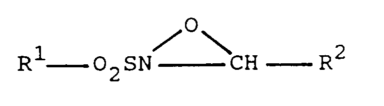 wherein R¹ is phenyl, phenyl substituted with a substituent which is stable under the conditions of reaction, C₃₋₇-cycloalkyl, camphoryl, or another cyclic or bicyclic radical which may carry alkyl, keto, or other substituents which are stable under the conditions of reaction, and wherein R² independently signifies hydrogen or the same radical as R¹, or wherein R¹ and R² together form a cyclic or bicyclic radical;
to produce an astaxanthin dihemiaminal having the formula 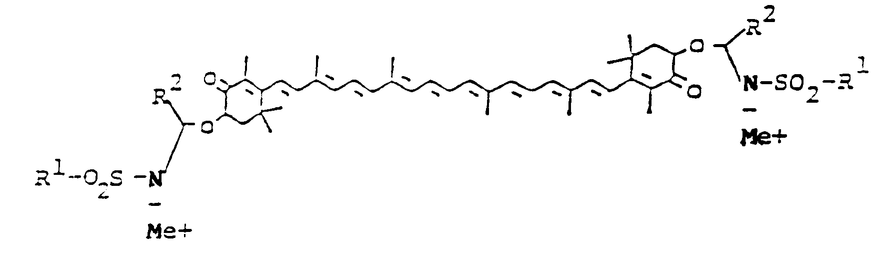 wherein R¹, R² and Me are as defined above,
and decomposing the astaxanthin dihemiaminal to obtain the astaxanthin.

2. The process of claim 1, wherein both R¹ and R² are phenyl.

3. The process of claim 1, wherein R¹ and R² together are camphoryl.

4. The process of claim 1, wherein the oxidation is carried out in the presence of an organic solvent which is nonreactive with the reactants and reaction products under the conditions of reaction.

5. The process of claim 4, wherein the solvent is tetrahydrofuran, dioxane, or an aromatic solvent.

6. The process of claim 4, wherein the oxidation is carried out at a temperature between -78°C and -20°C.

7. The process of claim 1, wherein the oxidation is carried out in an inert atmosphere.

8. The process of claim 1, wherein decomposition is carried out with a proton donor at a temperature up to 30°C.

9. The process of claim 2, wherein the decomposition is carried out in the presence of an organic solvent which is non-reactive with the reactants and reaction products under the conditions of reaction and at a temperature up to 30°C and employing a proton donor.

10. The process of claim 8, wherein the proton donor is acetic acid or ammonium chloride.

11. The process of claim 2 or 3, wherein the decomposition is carried out in the presence of an organic solvent which is non-reactive with the reactants and reaction products under the conditions of reaction at a temperature up to 30°C and in the presence of a proton donor, and wherein the oxidation is carried out in the presence of an inert gas at a temperature between -78°C and -20°C and in the presence of an organic solvent which is non-reactive with the reactants and reaction products under the conditions of reaction.

12. The process of claim 1, wherein an excess of the oxidant is employed.

13. An astaxanthin dihemiaminal having the formula 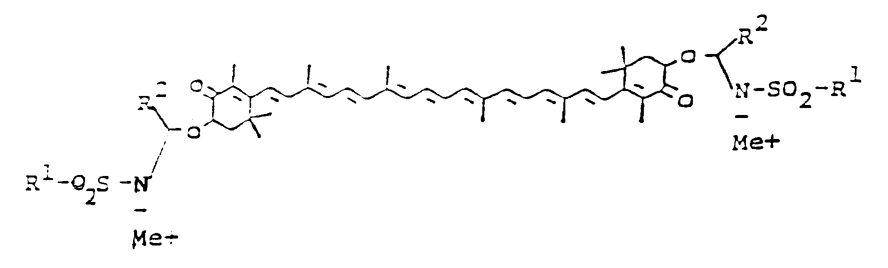 wherein R¹ is phenyl, phenyl substituted with a substituent which is stable under the conditions of the process defined in claim 1, C₃₋₇-cycloalkyl, camphoryl, or another cyclic or bicyclic radical which may carry alkyl, keto or other substituents which are stable under the conditions of the process defined in claim 1, and wherein R² independently signifies hydrogen or the same radical as R¹, or wherein R¹ and R² together form a cyclic or bicyclic radical, and Me is an alkali metal.

14. A compound of claim 13, wherein both R¹ and R² are phenyl.

15. A compound of claim 13, wherein R¹ and R² together are camphoryl.

16. A process for preparing a composition containing a compound of any of claims 13 to 15 comprising mixing said compound and an organic solvent.

17. The process of claim 16, wherein the solvent is tetrahydrofuran, dioxane, or an aromatic solvent.

## Patentansprüche (Patentansprüche für folgende(n) Vertragsstaat(en): AT, BE, CH, DE, DK, FR, GB, IT, LI, LU, NL, SE)

1. Verfahren zur Herstellung von Astaxanthin mit der Formel 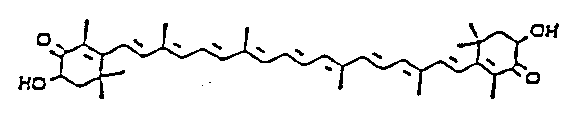 umfassend die Schritte:
Behandeln von Canthaxanthin mit einer Alkalimetallbase, um ein Canthaxanthinalkalimetalldienolat der Formel 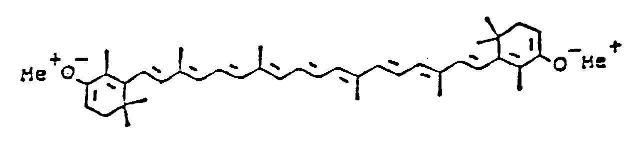 herzustellen, worin Me ein Alkalimetall ist,
Behandeln des Canthaxanthinalkalimetalldienolats mit einer Verbindung mit der Formel 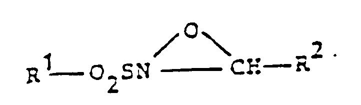 worin R¹ Phenyl, Phenyl, das mit einem Substituenten substituiert ist, der unter den Reaktionsbedingungen stabil ist, C₃₋₇-Cycloalkyl, Camphoryl oder ein anderer cyclischer oder bicyclischer Rest ist, welcher Alkyl-, Keto- oder andere Substituenten, die unter den Reaktionsbedingungen stabil sind, tragen kann, und worin R² unabhängig Wasserstoff oder den gleichen Rest wie R¹ bedeutet, oder worin R¹ und R² zusammen einen cyclischen oder bicyclischen Rest bilden;
um ein Astaxanthindihemiaminal mit der Formel 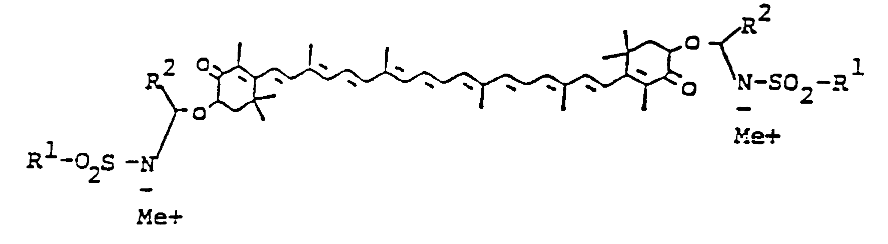 herzustellen, worin R¹, R² und Me wie vorstehend definiert sind, und
Zersetzen des Astaxanthindihemiaminals, um das Astaxanthin zu erhalten.

2. Verfahren nach Anspruch 1, worin sowohl R¹ als auch R² Phenyl sind.

3. Verfahren nach Anspruch 1, worin R¹ und R² zusammen Camphoryl sind.

4. Verfahren nach Anspruch 1, worin die Oxidation in Gegenwart eines organischen Lösungsmittels ausgeführt wird, welches mit den Reaktanten und Reaktionsprodukten unter den Reaktionsbedingungen nicht reagiert.

5. Verfahren nach Anspruch 4, worin das Lösungsmittel Tetrahydrofuran, Dioxan oder ein aromatisches Lösungsmittel ist.

6. Verfahren nach Anspruch 4, worin die Oxidation bei einer Temperatur zwischen -78°C und -20°C durchgeführt wird.

7. Verfahren nach Anspruch 1, worin die Oxidation in einer Inertatmosphäre durchgeführt wird.

8. Verfahren nach Anspruch 1, worin die Zersetzung mit einem Protonendonor bei einer Temperatur bis zu 30°C durchgeführt wird.

9. Verfahren nach Anspruch 2, worin die Zersetzung in Gegenwart eines organischen Lösungsmittels, welches mit den Reaktanten und Reaktionsprodukten unter den Reaktionsbedingungen nicht reagiert, und bei einer Temperatur bis zu 30°C und unter Verwendung eines Protonendonors durchgeführt wird.

10. Verfahren nach Anspruch 8, worin der Protonendonor Essigsäure oder Ammoniumchlorid ist.

11. Verfahren nach Anspruch 2 oder 3, worin die Zersetzung in Gegenwart eines organischen Lösungsmittels, welches mit den Reaktanten und Reaktionsprodukten unter den Reaktionsbedingungen nicht reagiert, bei einer Temperatur bis zu 30°C und in Gegenwart eines Protonendonors durchgeführt wird, und worin die Oxidation in Gegenwart eines Inertgases bei einer Temperatur zwischen -78°C und -20°C und in Gegenwart eines organischen Lösungsmittels, welches mit den Reaktanten und Reaktionsprodukten unter den Reaktionsbedingungen nicht reagiert, durchgeführt wird.

12. Verfahren nach Anspruch 1, worin ein Überschuß des Oxidationsmittels eingesetzt wird.

13. Astaxanthindihemiaminal mit der Formel 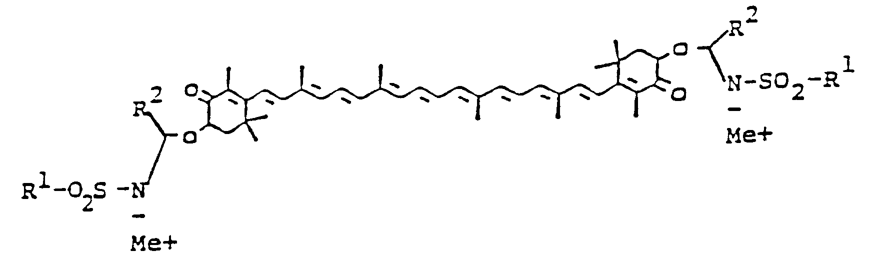 worin R¹ Phenyl, Phenyl, das mit einem Substituenten substituiert ist, der unter den Bedingungen des Verfahrens, wie es in Anspruch 1 definiert ist, stabil ist, C₃₋₇-Cycloalkyl, Camphoryl oder ein anderer cyclischer oder bicyclischer Rest ist, welcher Alkyl-, Keto- oder andere Substituenten, die unter den Bedingungen des Verfahrens, wie es in Anspruch 1 definiert ist, stabil sind, tragen kann, und worin R² unabhängig Wasserstoff oder den gleichen Rest wie R¹ bedeutet, oder worin R¹ und R² zusammen einen cyclischen oder bicyclischen Rest bilden, und Me ein Alkalimetall ist.

14. Verbindung nach Anspruch 13, worin sowohl R¹ als auch R² Phenyl sind.

15. Verbindung nach Anspruch 13, worin R¹ und R² zusammen Camphoryl sind.

16. Zusammensetzung, enthaltend eine Verbindung nach einem der Ansprüche 13-15, in einem organischen Lösungsmittel.

17. Zusammensetzung nach Anspruch 16, worin das Lösungsmittel Tetrahydrofuran, Dioxan oder ein aromatisches Lösungsmittel ist.

## Patentansprüche (Patentansprüche für folgende(n) Vertragsstaat(en): ES)

1. Verfahren zur Herstellung von Astaxanthin mit der Formel 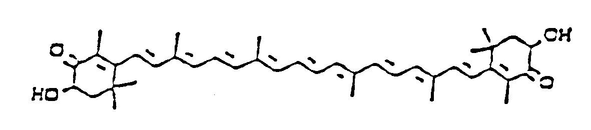 umfassend die Schritte:
Behandeln von Canthaxanthin mit einer Alkalimetallbase, um ein Canthaxanthinalkalimetalldienolat der Formel 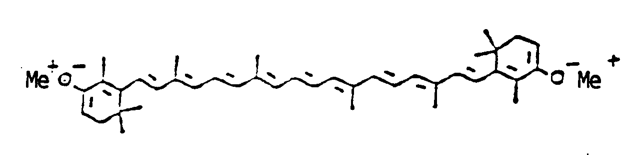 herzustellen, worin Me ein Alkalimetall ist,
Behandeln des Canthaxanthinalkalimetalldienolats mit einer Verbindung mit der Formel 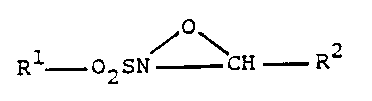 worin R¹ Phenyl, Phenyl, das mit einem Substituenten substituiert ist, der unter den Reaktionsbedingungen stabil ist, C₃₋₇-Cycloalkyl, Camphoryl oder ein anderer cyclischer oder bicyclischer Rest ist, welcher Alkyl-, Keto- oder andere Substituenten, die unter den Reaktionsbedingungen stabil sind, tragen kann, und worin R² unabhängig Wasserstoff oder den gleichen Rest wie R¹ bedeutet, oder worin R¹ und R² zusammen einen cyclischen oder bicyclischen Rest bilden;
um ein Astaxanthindihemiaminal mit der Formel 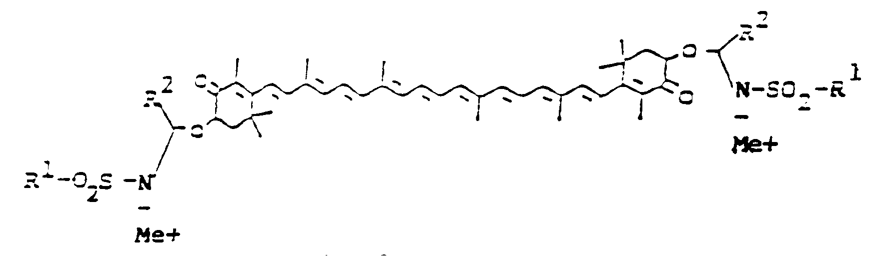 herzustellen, worin R¹, R² und Me wie vorstehend definiert sind,
und Zersetzen des Astaxanthindihemiaminals, um das Astaxanthin zu erhalten.

2. Verfahren nach Anspruch 1, worin sowohl R¹ als auch R² Phenyl sind.

3. Verfahren nach Anspruch 1, worin R¹ und R² zusammen Camphoryl sind.

4. Verfahren nach Anspruch 1, worin die Oxidation in Gegenwart eines organischen Lösungsmittels ausgeführt wird, welches mit den Reaktanten und Reaktionsprodukten unter den Reaktionsbedingungen nicht reagiert.

5. Verfahren nach Anspruch 4, worin das Lösungsmittel Tetrahydrofuran, Dioxan oder ein aromatisches Lösungsmittel ist.

6. Verfahren nach Anspruch 4, worin die Oxidation bei einer Temperatur zwischen -78°C und -20°C durchgeführt wird.

7. Verfahren nach Anspruch 1, worin die Oxidation in einer Inertatmosphäre durchgeführt wird.

8. Verfahren nach Anspruch 1, worin die Zersetzung mit einem Protonendonor bei einer Temperatur bis zu 30°C durchgeführt wird.

9. Verfahren nach Anspruch 2, worin die Zersetzung in Gegenwart eines organischen Lösungsmittels, welches mit den Reaktanten und Reaktionsprodukten unter den Reaktionsbedingungen nicht reagiert, und bei einer Temperatur bis zu 30°C und unter Verwendung eines Protonendonors durchgeführt wird.

10. Verfahren nach Anspruch 8, worin der Protonendonor Essigsäure oder Ammoniumchlorid ist.

11. Verfahren nach Anspruch 2 oder 3, worin die Zersetzung in Gegenwart eines organischen Lösungsmittels, welches mit den Reaktanten und Reaktionsprodukten unter den Reaktionsbedingungen nicht reagiert, bei einer Temperatur bis zu 30°C und in Gegenwart eines Protonendonors durchgeführt wird, und worin die Oxidation in Gegenwart eines Inertgases bei einer Temperatur zwischen -78°C und -20°C und in Gegenwart eines organischen Lösungsmittels, welches mit den Reaktanten und Reaktionsprodukten unter den Reaktionsbedingungen nicht reagiert, durchgeführt wird.

12. Verfahren nach Anspruch 1, worin ein Überschuß des Oxidationsmittels eingesetzt wird.

13. Verfahren zum Herstellen eines Astaxanthindihemiaminals mit der Formel 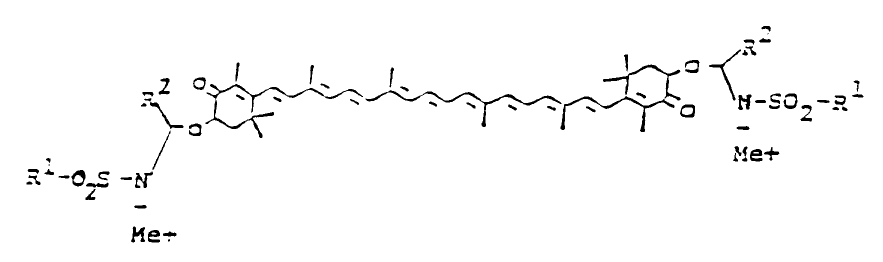 worin R¹ Phenyl, Phenyl, das mit einem Substituenten substituiert ist, der unter den Bedingungen des Verfahrens, wie es in Anspruch 1 definiert ist, stabil ist, C₃₋₇-Cycloalkyl, Camphoryl oder ein anderer cyclischer oder bicyclischer Rest ist, welcher Alkyl-, Keto- oder andere Substituenten, die unter den Bedingungen des Verfahrens, wie es in Anspruch 1 definiert ist, stabil sind, tragen kann, und worin R² unabhängig Wasserstoff oder den gleichen Rest wie R¹ bedeutet, oder worin R¹ und R² zusammen einen cyclischen oder bicyclischen Rest bilden und Me ein Alkalimetall ist,
umfassend die Schritte
Behandeln von Canthaxanthin mit einer Alkalimetallbase, um ein Canthaxanthinalkalimetalldienolat der Formel 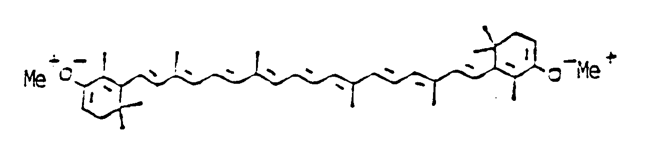 herzustellen, worin Me wie vorstehend definiert ist, und
Behandeln des Canthaxanthinalkalimetalldienolats mit einer Verbindung mit der Formel 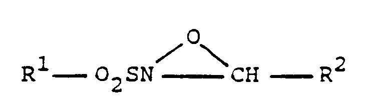 worin R¹ und R² wie vorstehend definiert sind, um das Astaxanthindihemiaminal herzustellen.

14. Verfahren nach Anspruch 13, worin sowohl R¹ als auch R² Phenyl sind.

15. Verfahren nach Anspruch 13, worin R¹ und R² zusammen Camphoryl sind.

16. Verfahren zum Herstellen einer Zusammensetzung, enthaltend eine Verbindung, die nach einem der Ansprüche 13-15 hergestellt ist, umfassend das Vermischen der Verbindung und eines organischen Lösungsmittels.

17. Verfahren nach Anspruch 16, worin das Lösungsmittel Tetrahydrofuran, Dioxan oder ein aromatisches Lösungsmittel ist.

## Patentansprüche (Patentansprüche für folgende(n) Vertragsstaat(en): GR)

1. Verfahren zur Herstellung von Astaxanthin mit der Formel 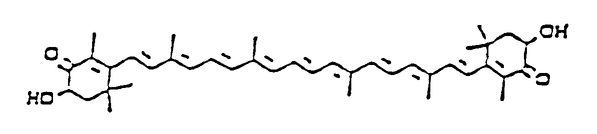 umfassend die Schritte:
Behandeln von Canthaxanthin mit einer Alkalimetallbase, um ein Canthaxanthinalkalimetalldienolat der Formel 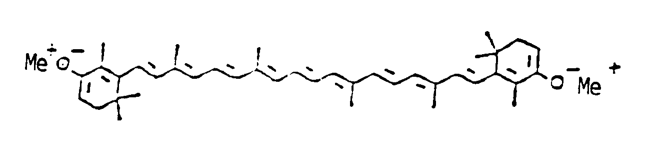 herzustellen, worin Me ein Alkalimetall ist,
Behandeln des Canthaxanthinalkalimetalldienolats mit einer Verbindung mit der Formel 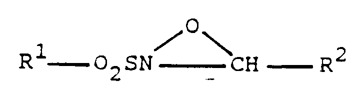 worin R¹ Phenyl, Phenyl, das mit einem Substituenten substituiert ist, der unter den Reaktionsbedingungen stabil ist, C₃₋₇-Cycloalkyl, Camphoryl oder ein anderer cyclischer oder bicyclischer Rest ist, welcher Alkyl-, Keto- oder andere Substituenten, die unter den Reaktionsbedingungen stabil sind, tragen kann, und worin R² unabhängig Wasserstoff oder den gleichen Rest wie R¹ bedeutet, oder worin R¹ und R² zusammen einen cyclischen oder bicyclischen Rest bilden;
um ein Astaxanthindihemiaminal mit der Formel 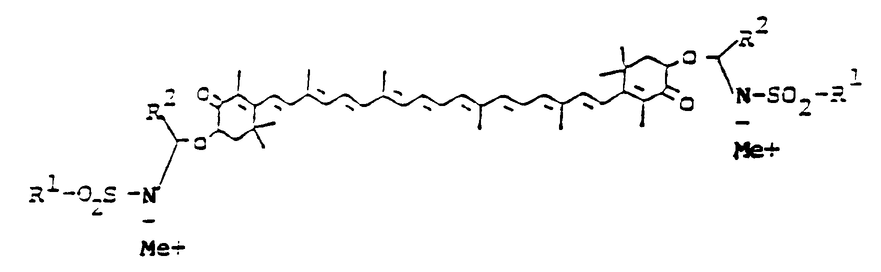 herzustellen, worin R¹, R² und Me wie vorstehend definiert sind,
und Zersetzen des Astaxanthindihemiaminals, um das Astaxanthin zu erhalten.

2. Verfahren nach Anspruch 1, worin sowohl R¹ als auch R² Phenyl sind.

3. Verfahren nach Anspruch 1, worin R¹ und R² zusammen Camphoryl sind.

4. Verfahren nach Anspruch 1, worin die Oxidation in Gegenwart eines organischen Lösungsmittels ausgeführt wird, welches mit den Reaktanten und Reaktionsprodukten unter den Reaktionsbedingungen nicht reagiert.

5. Verfahren nach Anspruch 4, worin das Lösungsmittel Tetrahydrofuran, Dioxan oder ein aromatisches Lösungsmittel ist.

6. Verfahren nach Anspruch 4, worin die Oxidation bei einer Temperatur zwischen -78°C und -20°C durchgeführt wird.

7. Verfahren nach Anspruch 1, worin die Oxidation in einer Inertatmosphäre durchgeführt wird.

8. Verfahren nach Anspruch 1, worin die Zersetzung mit einem Protonendonor bei einer Temperatur bis zu 30°C durchgeführt wird.

9. Verfahren nach Anspruch 2, worin die Zersetzung in Gegenwart eines organischen Lösungsmittels, welches mit den Reaktanten und Reaktionsprodukten unter den Reaktionsbedingungen nicht reagiert, und bei einer Temperatur bis zu 30°C und unter Verwendung eines Protonendonors durchgeführt wird.

10. Verfahren nach Anspruch 8, worin der Protonendonor Essigsäure oder Ammoniumchlorid ist.

11. Verfahren nach Anspruch 2 oder 3, worin die Zersetzung in Gegenwart eines organischen Lösungsmittels, welches mit den Reaktanten und Reaktionsprodukten unter den Reaktionsbedingungen nicht reagiert, bei einer Temperatur bis zu 30°C und in Gegenwart eines Protonendonors durchgeführt wird, und worin die Oxidation in Gegenwart eines Inertgases bei einer Temperatur zwischen -78°C und -20°C und in Gegenwart eines organischen Lösungsmittels, welches mit den Reaktanten und Reaktionsprodukten unter den Reaktionsbedingungen nicht reagiert, durchgeführt wird.

12. Verfahren nach Anspruch 1, worin ein Überschuß des Oxidationsmittels eingesetzt wird.

13. Astaxanthindihemiaminal mit der Formel 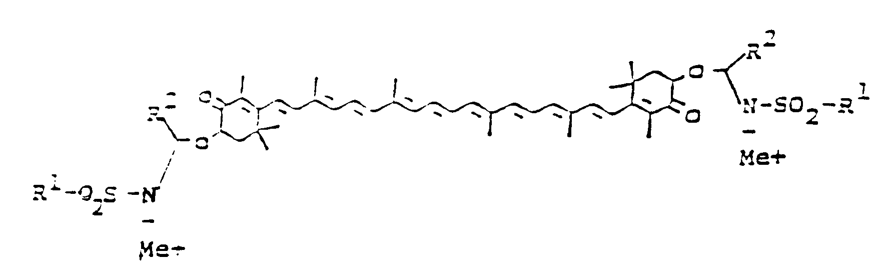 worin R¹ Phenyl, Phenyl, das mit einem Substituenten substituiert ist, der unter den Bedingungen des Verfahrens, wie es in Anspruch 1 definiert ist, stabil ist, C₃₋₇-Cycloalkyl, Camphoryl oder ein anderer cyclischer oder bicyclischer Rest ist, welcher Alkyl-, Keto- oder andere Substituenten, die unter den Bedingungen des Verfahrens, wie es in Anspruch 1 definiert ist, stabil sind, tragen kann, und worin R² unabhängig Wasserstoff oder den gleichen Rest wie R¹ bedeutet, oder worin R¹ und R² zusammen einen cyclischen oder bicyclischen Rest bilden, und Me ein Alkalimetall ist.

14. Verbindung nach Anspruch 13, worin sowohl R¹ als auch R² Phenyl sind.

15. Verbindung nach Anspruch 13, worin R¹ und R² zusammen Camphoryl sind.

16. Verfahren zum Herstellen einer Zusammensetzung, enthaltend eine Verbindung nach einem der Ansprüche 13-15, umfassend das Vermischen der Verbindung und eines organischen Lösungsmittels.

17. Verfahren nach Anspruch 16, worin das Lösungsmittel Tetrahydrofuran, Dioxan oder ein aromatisches Lösungsmittel ist.

## Revendications (Revendications pour l'(les) Etat(s) contractant(s) suivant(s): AT, BE, CH, DE, DK, FR, GB, IT, LI, LU, NL, SE)

1. Procédé de préparation d'astaxanthine de formule 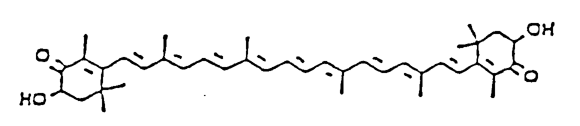 comprenant les étapes de
traitement de la canthaxanthine avec une base de métal alcalin pour produire un diénolate de métal alcalin-canthaxanthine de formule 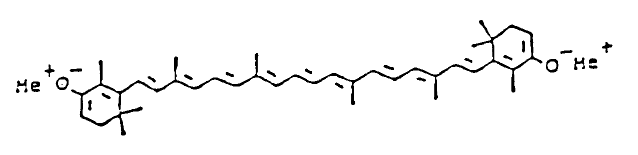 dans laquelle
Me est un métal alcalin,
traitement du diénolate de métal alcalin-canthaxanthine avec un composé de formule 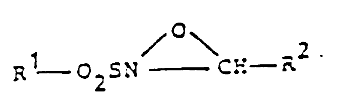 dans laquelle R¹ est un phényle, un phényle substitué par un substituant qui est stable dans les conditions de la réaction, un cycloalkyle en C₃ à C₇ , un camphoroyle, ou un autre radical cyclique ou bicyclique qui peut porter des substituants alkyle, céto ou autres qui sont stables dans les conditions de la réaction, et où R² représente indépendamment un hydrogène ou le même radical que R¹, ou bien où R¹ et R² forment ensemble un radical cyclique ou bicyclique;
pour produire un dihémiaminal d'astaxanthine de formule 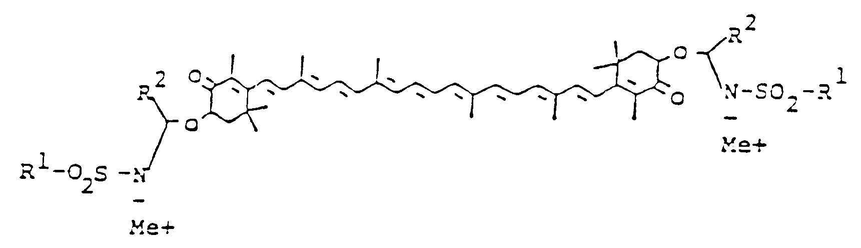 dans laquelle R¹ , R² et Me sont tels que définis ci-dessus,
et décomposition du dihémiaminal d'astaxanthine pour obtenir l'astaxanthine.

2. Procédé de la revendication 1, dans lequel R¹ comme R² représentent un phényle.

3. Procédé de la revendication 1, dans lequel R¹ et R² représentent ensemble un camphoroyle.

4. Procédé de la revendication 1, dans lequel l'oxydation est conduite en présence d'un solvant organique qui ne réagit pas avec les réactifs et les produits de la réaction dans les conditions de la réaction.

5. Procédé de la revendication 4, dans lequel le solvant est le tétrahydrofuranne, le dioxanne ou un solvant aromatique.

6. Procédé de la revendication 4, dans lequel l'oxydation est conduite à une température comprise entre -78°C et -20°C.

7. Procédé de la revendication 1, dans lequel l'oxydation est conduite dans une atmosphère inerte.

8. Procédé de la revendication 1, dans lequel la décomposition est conduite avec un donneur de protons à une température allant jusqu'à 30°C.

9. Procédé de la revendication 2, dans lequel la décomposition est conduite en présence d'un solvant organique qui ne réagit pas avec les réactifs et les produits de la réaction dans les conditions de la réaction et à une température allant jusqu'à 30°C et en employant un donneur de protons.

10. Procédé de la revendication 8, dans lequel le donneur de protons est l'acide acétique ou le chlorure d'ammonium.

11. Procédé de la revendication 2 ou 3, dans lequel la décomposition est conduite en présence d'un solvant organique qui ne réagit pas avec les réactifs et les produits de la réaction dans les conditions de la réaction à une température allant jusqu'à 30°C et en présence d'un donneur de protons, et où l'oxydation est conduite en présence d'un gaz inerte à une température comprise entre -78°C et -20°C et en présence d'un solvant organique qui ne réagit pas avec les réactifs et les produits de la réaction dans les conditions de la réaction.

12. Procédé de la revendication 1, dans lequel on emploie un excès de l'oxydant.

13. Dihémiaminal d'astaxanthine de formule 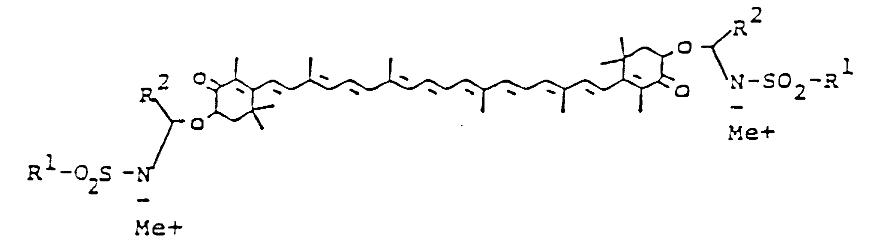 dans laquelle R¹ est un phényle, un phényle substitué par un substituant qui est stable dans les conditions du procédé défini dans la revendication 1, un cycloalkyle en C₃ à C₇, un camphoroyle ou un autre radical cyclique ou bicyclique qui peut porter des substituants alkyle, céto ou autres qui sont stables dans les conditions du procédé défini dans la revendication 1, et où R² représente indépendamment un hydrogène ou le même radical que R¹, ou bien où R¹ et R² forment ensemble un radical cyclique ou bicyclique, et Me est un métal alcalin.

14. Composé de la revendication 13, dans lequel tant R¹ que R² représentent un phényle.

15. Composé de la revendication 13, dans lequel R¹ et R² représentent ensemble un camphoroyle.

16. Composition contenant un composé de l'une quelconque des revendications 13 à 15, dans un solvant organique.

17. Composition de la revendication 16, dans laquelle le solvant est le tétrahydrofuranne, le dioxanne ou un solvant aromatique.

## Revendications (Revendications pour l'(les) Etat(s) contractant(s) suivant(s): ES)

1. Procédé de préparation d'astaxanthine de formule 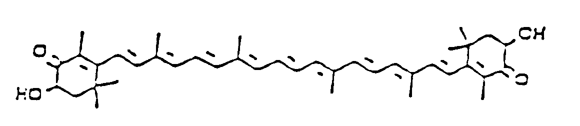 comprenant les étapes de
traitement de la canthaxanthine avec une base de métal alcalin pour produire un diénolate de métal alcalin-canthaxanthine de formule 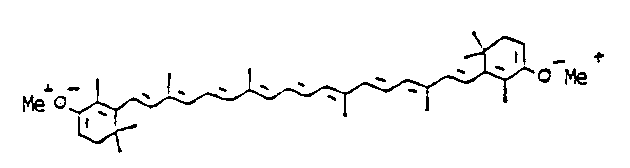 dans laquelle
Me est un métal alcalin,
traitement du diénolate de métal alcalin-canthaxanthine avec un composé de formule 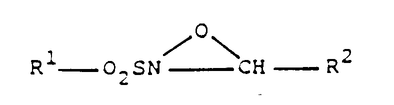 dans laquelle R¹ est un phényle, un phényle substitué par un substituant qui est stable dans les conditions de la réaction, un cycloalkyle en C₃ à C₇ , un camphoroyle, ou un autre radical cyclique ou bicyclique qui peut porter des substituants alkyle, céto ou autres qui sont stables dans les conditions de la réaction, et où R² représente indépendamment un hydrogène ou le même radical que R¹, ou bien où R¹ et R² forment ensemble un radical cyclique ou bicyclique;
pour produire un dihémiaminal d'astaxanthine de formule 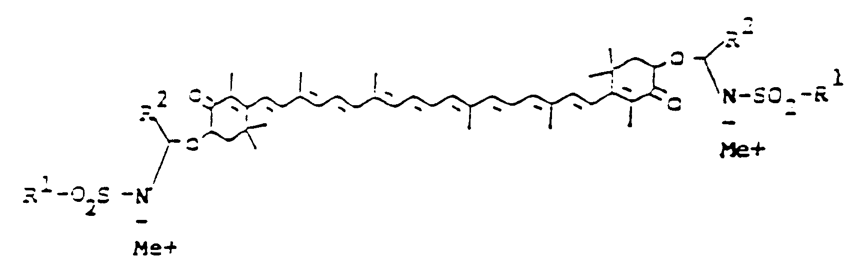 dans laquelle R¹ , R² et Me sont tels que définis ci-dessus,
et décomposition du dihémiaminal d'astaxanthine pour obtenir l'astaxanthine.

2. Procédé de la revendication 1, dans lequel R¹ comme R² représentent un phényle.

3. Procédé de la revendication 1, dans lequel R¹ et R² représentent ensemble un camphoroyle.

4. Procédé de la revendication 1, dans lequel l'oxydation est conduite en présence d'un solvant organique qui ne réagit pas avec les réactifs et les produits de la réaction dans les conditions de la réaction.

5. Procédé de la revendication 4, dans lequel le solvant est le tétrahydrofuranne, le dioxanne ou un solvant aromatique.

6. Procédé de la revendication 4, dans lequel l'oxydation est conduite à une température comprise entre -78°C et -20°C.

7. Procédé de la revendication 1, dans lequel l'oxydation est conduite dans une atmosphère inerte.

8. Procédé de la revendication 1, dans lequel la décomposition est conduite avec un donneur de protons à une température allant jusqu'à 30°C.

9. Procédé de la revendication 2, dans lequel la décomposition est conduite en présence d'un solvant organique qui ne réagit pas avec les réactifs et les produits de la réaction dans les conditions de la réaction et à une température allant jusqu'à 30°C et en employant un donneur de protons.

10. Procédé de la revendication 8, dans lequel le donneur de protons est l'acide acétique ou le chlorure d'ammonium.

11. Procédé de la revendication 2 ou 3, dans lequel la décomposition est conduite en présence d'un solvant organique qui ne réagit pas avec les réactifs et les produits de la réaction dans les conditions de la réaction à une température allant jusqu'à 30°C et en présence d'un donneur de protons, et où l'oxydation est conduite en présence d'un gaz inerte à une température comprise entre -78°C et -20°C et en présence d'un solvant organique qui ne réagit pas avec les réactifs et les produits de la réaction dans les conditions de la réaction.

12. Procédé de la revendication 1, dans lequel on emploie un excès de l'oxydant.

13. Procédé de préparation d'un dihémiaminal d'astaxanthine de formule 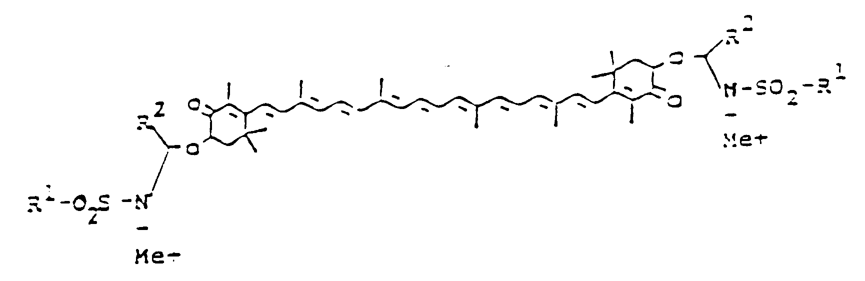 dans laquelle R¹ est un phényle, un phényle substitué par un substituant qui est stable dans les conditions du procédé défini dans la revendication 1, un cycloalkyle en C₃ à C₇, un camphoroyle ou un autre radical cyclique ou bicyclique qui peut porter des substituants alkyle, céto ou autres qui sont stables dans les conditions du procédé défini dans la revendication 1, et où R² représente indépendamment un hydrogène ou le même radical que R¹, ou bien où R¹ et R² forment ensemble un radical cyclique ou bicyclique, et Me est un métal alcalin;
comprenant les étapes de:
traitement de la canthaxanthine avec une base de métal alcalin pour produire un diénolate de métal alcalin-canthaxanthine de formule 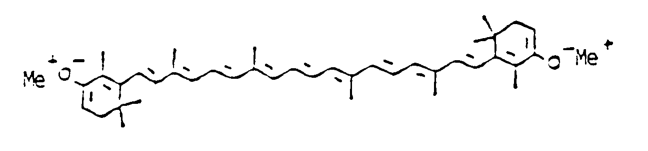 dans laquelle
Me est tel que défini ci-dessus, et
traitement du diénolate de métal alcalin-canthaxanthine avec un composé de formule 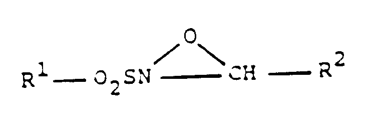 dans laquelle R¹ et R² sont tels que définis ci-dessus pour produire le dihémiaminal d'astaxanthine.

14. Procédé de la revendication 13, dans lequel tant R¹ que R² représentent un phényle.

15. Composé de la revendication 13, dans lequel R¹ et R² représentent ensemble un camphoroyle.

16. Procédé de préparation d'une composition contenant un composé préparé selon l'une quelconque des revendications 13 à 15, dans lequel on mélange ledit composé dans un solvant organique.

17. Procédé de la revendication 16, dans lequel le solvant est le tétrahydrofuranne, le dioxanne ou un solvant aromatique.

## Revendications (Revendications pour l'(les) Etat(s) contractant(s) suivant(s): GR)

1. Procédé de préparation d'astaxanthine de formule 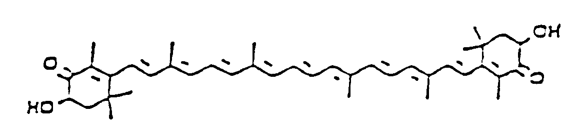 comprenant les étapes de
traitement de la canthaxanthine avec une base de métal alcalin pour produire un diénolate de métal alcalin-canthaxanthine de formule 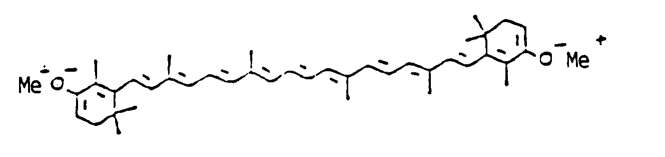 dans laquelle
Me est un métal alcalin,
traitement du diénolate de métal alcalin-canthaxanthine avec un composé de formule 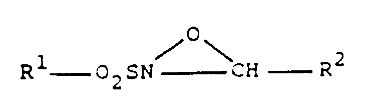 dans laquelle R¹ est un phényle, un phényle substitué par un substituant qui est stable dans les conditions de la réaction, un cycloalkyle en C₃ à C₇ , un camphoroyle, ou un autre radical cyclique ou bicyclique qui peut porter des substituants alkyle, céto ou autres qui sont stables dans les conditions de la réaction, et où R² représente indépendamment un hydrogène ou le même radical que R¹, ou bien où R¹ et R² forment ensemble un radical cyclique ou bicyclique;
pour produire un dihémiaminal d'astaxanthine de formule 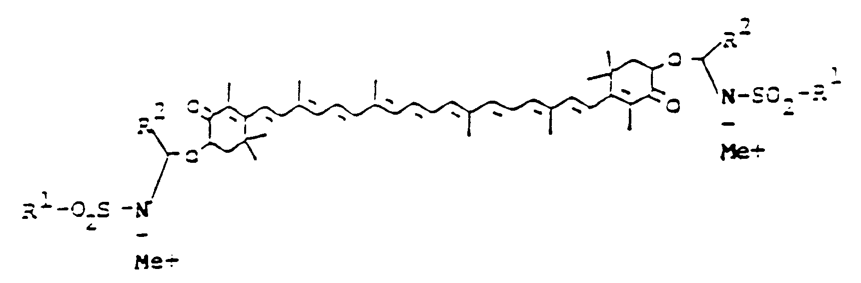 dans laquelle R¹ , R² et Me sont tels que définis ci-dessus,
et décomposition du dihémiaminal d'astaxanthine pour obtenir l'astaxanthine.

2. Procédé de la revendication 1, dans lequel R¹ comme R² représentent un phényle.

3. Procédé de la revendication 1, dans lequel R¹ et R² représentent ensemble un camphoroyle.

4. Procédé de la revendication 1, dans lequel l'oxydation est conduite en présence d'un solvant organique qui ne réagit pas avec les réactifs et les produits de la réaction dans les conditions de la réaction.

5. Procédé de la revendication 4, dans lequel le solvant est le tétrahydrofuranne, le dioxanne ou un solvant aromatique.

6. Procédé de la revendication 4, dans lequel l'oxydation est conduite à une température comprise entre -78°C et -20°C.

7. Procédé de la revendication 1, dans lequel l'oxydation est conduite dans une atmosphère inerte.

8. Procédé de la revendication 1, dans lequel la décomposition est conduite avec un donneur de protons à une température allant jusqu'à 30°C.

9. Procédé de la revendication 2, dans lequel la décomposition est conduite en présence d'un solvant organique qui ne réagit pas avec les réactifs et les produits de la réaction dans les conditions de la réaction et à une température allant jusqu'à 30°C et en employant un donneur de protons.

10. Procédé de la revendication 8, dans lequel le donneur de protons est l'acide acétique ou le chlorure d'ammonium.

11. Procédé de la revendication 2 ou 3, dans lequel la décomposition est conduite en présence d'un solvant organique qui ne réagit pas avec les réactifs et les produits de la réaction dans les conditions de la réaction à une température allant jusqu'à 30°C et en présence d'un donneur de protons, et où l'oxydation est conduite en présence d'un gaz inerte à une température comprise entre -78°C et -20°C et en présence d'un solvant organique qui ne réagit pas avec les réactifs et les produits de la réaction dans les conditions de la réaction.

12. Procédé de la revendication 1, dans lequel on emploie un excès de l'oxydant.

13. Dihémiaminal d'astaxanthine de formule 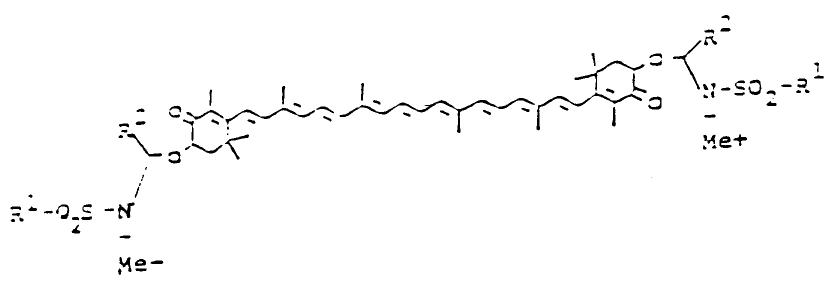 dans laquelle R¹ est un phényle, un phényle substitué par un substituant qui est stable dans les conditions du procédé défini dans la revendication 1, un cycloalkyle en C₃ à C₇, un camphoroyle ou un autre radical cyclique ou bicyclique qui peut porter des substituants alkyle, céto ou autres qui sont stables dans les conditions du procédé défini dans la revendication 1, et où R² représente indépendamment un hydrogène ou le même radical que R¹, ou bien où R¹ et R² forment ensemble un radical cyclique ou bicyclique, et Me est un métal alcalin.

14. Composé de la revendication 13, dans lequel tant R¹ que R² représentent un phényle.

15. Composé de la revendication 13, dans lequel R¹ et R² représentent ensemble un camphoroyle.

16. Procédé de préparation d'une composition contenant un composé préparé selon l'une quelconque des revendications 13 à 15, dans lequel on mélange ledit composé dans un solvant organique.

17. Procédé de la revendication 16, dans lequel le solvant est le tétrahydrofuranne, le dioxanne ou un solvant aromatique.
